# EUROPEAN PATENT SPECIFICATION

(11) **EP 3 687 393 B1**
(45) Date of publication and mention of the grant of the patent: **01.11.2023**
(21) Application number: 18789739.2
(22) Date of filing: 26.09.2018
(51) Int. Cl.: A61B 5/00, A61B 5/25

(54) **DEVICE AND SYSTEM FOR PROVIDING PHYSIOLOGICAL DATA MONITORING OF PATIENTS**
VORRICHTUNG UND SYSTEM ZUR ÜBERWACHUNG PHYSIOLOGISCHER DATEN VON PATIENTEN
DISPOSITIF ET SYSTÈME POUR ASSURER UNE SURVEILLANCE DES DONNÉES PHYSIOLOGIQUES DE PATIENTS

(30) Priority: 27.09.2017 US 201762564104 P
(43) Date of publication of application: 05.08.2020
(73) Proprietor: Drägerwerk AG & Co. KGaA, 23558 Lübeck (DE)
(72) Inventor: YOMTOV, Barry, Marblehead, Massachusetts 01945 (US)
(74) Representative: Haseltine Lake Kempner LLP
(86) International application number: PCT/US2018/052803
(87) International publication number: WO 2019/067507

(56) References cited:
- WO-A1-2016/181321
- US-A- 5 788 633
- US-A1- 2006 264 767
- US-A1- 2011 125 040
- US-A1- 2013 225 967
- US-A1- 2015 022 372
- US-A1- 2015 087 921
- US-A1- 2017 100 046

## Description

### Technical Field

The subject matter of the present disclosure relates generally to patient monitoring devices used for collecting physiological data.

### Background

Patient monitoring devices are essential medical devices that provide vital physiological data to clinicians and caregivers for the care of patients. However, patient monitoring can present challenges both inside and outside hospital environments. For example, patients that are admitted to a healthcare facility may require continuous physiological monitoring, and this continual physiological monitoring can be a data intensive task. These challenges can be accentuated when the patients being monitored are ambulatory (i.e., moveable) because the devices used for monitoring patient parameters are also required to be ambulatory (i.e., moveable) so that patients are not confined to a particular bed or to a particular care unit.

Healthcare facilities have been outfitted with a network of wireless access points that enable wireless communication between a central monitoring station and patient monitoring devices. With the implementation of wireless data communications, the ability to remotely monitor patients has expanded the use of ambulatory patient monitoring devices used for monitoring physiological data. However, even with ambulatory patient monitoring devices, there are still challenges regarding device performance, patient compliance, and quality of life for patients when these devices are used over long periods of time.

Additionally, with hospital enterprises now expanding to alternate lower acuity care settings such as rehabilitation centers, the requirements for continuous physiological data monitoring has become more focused on quality of life when considering patients are more mobile (ambulatory) and the physiological monitors are operating 24 hours a day.

Thus, it would be advantageous and an improvement over conventional patient monitoring systems to provide a patient worn physiological monitoring device or system for ambulatory patients that improve device performance, patient compliance, quality of life for patients, and overall patient outcomes during ambulatory activity associated with recuperation and rehabilitation.

The following documents disclose examples of patient monitoring systems:
- US 2011/125040 A1 describes an ECG monitoring system including a reusable battery-powered ECG monitor with an electrode attached to a patient for receiving ECG signals. A processor analyzes the received ECG signals for predefined arrhythmia. If an arrhythmia is detected, a wireless transceiver in the ECG monitor transmits the event information and an ECG strip to a cellphone handset.
- US 2017/100046 A1 describes a wearable wireless 12-channel electrocardiogram system including: a wearable integrated electrocardiogram measurement device including a single electrode sheet having 10 electrodes and capable of being attached to a chest, and a micro-electrocardiogram measurement module detachably attached to and integrated with the electrode sheet, and configured to receive electrical signals from the 10 electrodes, to process the received signals and to transmit the processed signals to the outside.
- US 2013/225967 A1 describes a self-contained electrocardiogram (EKG) device able to wirelessly communicate EKG data to a smart phone hosting an application that can receive and display the EKG data, as well as do some analysis to identify particular heart conditions and display that information, or data product. The smart phone receives the EKG data, makes it available to the application, and displays results produced by the application, which may include alarms or identification of specific heart problems.
- US 2015/087921 A1 describes physiological monitoring being provided through a wearable monitor including two components, a flexible extended wear electrode patch and a removable reusable monitor recorder. The wearable monitor can interoperate wirelessly with other physiology and activity sensors and mobile communications devices, to download monitoring data either in real-time or in batches.US 2006/264767 A1 describes a disposable programmable ECG sensor patch for the non-invasive detection of risk patterns according to programmed criteria. The patch is programmed by a medical professional to select one or more monitoring parameters for detection and alarm indication.
- US 2015/022372 A1 describes medical data acquisition systems and methods for monitoring and diagnosis. A system may include one or more electrodes configured to detect biological data and to convert the detected biological data into a signal. The system may also include a monitor recorder configured to receive the signal and store the detected biological data. Further, the system may include a transceiver configured to wirelessly communicate the biological data.
- WO 2016/181321 A1 describes an electrode device to acquire, process and transmit physiological signals. The device comprises a sensor patch comprising plurality of sensors/electrodes, configured to be in contact with a skin surface of a human body, to measure physiological of the patient. The wire-free module comprises a processing module to process one or more signals received from the plurality of sensors/ electrodes.
- US 5788633 A describes a ECG harness positioned on the chest of patient for making electrocardiographic measurements. The harness includes a strip of nonconductive film having a plurality of slots spaced along the length of the strip at selected anatomical positions. A connector
- terminal is formed at one edge of the strip for connection to a monitoring device.

### Summary

According to a first aspect, there is provided a physiological monitoring system in accordance with the appended independent claim 1. Further optional features are provided in the appended dependent claims. Described herein is a physiological monitoring system for providing monitoring of a patient including an electrocardiogram (ECG) module having an ECG microcontroller and a first plurality of electrodes worn on the patient; and a main module detachably worn by the patient and connected to the ECG module by a first communication connection. The ECG microcontroller is coupled to the first plurality of electrodes for receiving first physiological data gathered by the first plurality of electrodes, and the main module is configured to receive the first physiological data from the ECG module using the first communication connection.

The ECG microcontroller is further configured to analyze the first physiological data gathered by the first plurality of electrodes, identify one or more abnormal conditions of the patient, and transmit in real-time results of the analysis of the first physiological data and the identified abnormal conditions to the main module using the first communication connection. The first communication connection is a wireless communication connection.

Further described herein, the physiological monitoring system further comprising a detachable precordial electrode array including a second plurality of electrodes worn in a precordial location of the patient proximate to the ECG module. The second plurality of electrodes are configured to gather second physiological data, and the ECG module is connected to the detachable precordial electrode array by a second communication connection.

The ECG microcontroller is further configured to analyze the second physiological data gathered by the second plurality of electrodes, identify one or more abnormal conditions of the patient, and transmit in real-time results of the analysis of the second physiological data and the identified abnormal conditions to the main module using the first communication connection. The first communication connection is a wireless communication connection, and the second communication is a wired communication connection.

Also described herein, the main controller is further configured to transmit in real-time results of the analysis of the first physiological data or the second physiological data and the identified abnormal conditions using a first wireless protocol of the communication interface, or store the first physiological data or the second physiological data and the identified abnormal conditions in the on-board memory when the main module is unable to transmit the in real-time using the first wireless protocol of the communication interface.

The main controller is further configured transmit in real-time results of the analysis of the first physiological data or the second physiological data and the identified abnormal conditions using a second wireless protocol of the communication interface if a significant physiological event is identified from the analysis of the first physiological data or the second physiological data. The first wireless protocol is in accordance with WIFI or Bluetooth, whereas the second wireless protocol is in accordance with a cellular network.

Also described herein, the detachable precordial electrode array is connected to the main module by a third communication connection for transmitting the second physiological data gathered by the second plurality of electrodes to the main module. The main controller is configured to receive the second physiological data from the ECG module using the third communication connection, wherein the third connection is a wired connection.

Described herein, the detachable precordial electrode array includes a plurality of electrodes worn in a precordial location of the patient, and a communication connection for transmitting the physiological data gathered by the plurality of electrodes. The plurality of electrodes are formed in a flexible material pintegrated as a patch and having a bottom surface that is attachable to the patient for gathering the physiological data.

Described herein, a patch with the first plurality of electrodes is detachable from the data acquisition module and disposable, and the data acquisition module is re-useable.

Described herein, the data acquisition module and the patch with the first plurality of electrodes are integrated, and both the patch and the data acquisition module are disposable.

Described herein, the ECG module further comprises adjustment slots and a location of each of the first plurality of electrodes is adjustable within the adjustment slots.

Described herein, the detachable precordial array further comprises adjustment slots and a location of each of the second plurality of electrodes is adjustable within the adjustment slots.

### Brief Description of the Drawings

Fig. 1 is a block diagram of a main module for physiological monitoring according to an embodiment of the present disclosure;
Fig. 2 is an exemplary algorithm executed by the microcontrollers of the main module according to an embodiment of the present disclosure;
Fig. 3 is a block diagram of a wireless electrocardiogram (ECG) module according to an embodiment of the present disclosure;
Fig. 4 is an exemplary algorithm executed by a microcontroller of the wireless ECG module according to an embodiment of the present disclosure;
Fig. 5 is a diagram of a physiological monitoring system including the wireless ECG module and the main module according to an embodiment of the present disclosure;
Fig. 6 is a diagram of a physiological monitoring system including a precordial electrode array connected to the wireless ECG module according to an embodiment of the present disclosure;
Fig. 7A is a top view of the wireless ECG module according to an embodiment of the present disclosure;
Fig. 7B is side view of the wireless ECG module according to an embodiment of the present disclosure;
Fig. 8A is side view of the connection between the data acquisition module and the electrode patch of the wireless ECG module according to an embodiment of the present disclosure;
Fig. 8B is a bottom view of the electrode connections of the data acquisition module according to an embodiment of the present disclosure;
Fig. 9A is top view of the precordial electrode array with cable and in-line connector according to an embodiment of the present disclosure;
Fig. 9B is a cross-sectional view of the keyed twist lock on the cable of the precordial electrode array according to an embodiment of the present disclosure;
Fig. 10 is a side view of the connection between the data acquisition module and the precordial electrode array according to an embodiment of the present disclosure;
Figs. 11A and 11B are top views respectively of the adjustable electrode slots on the wireless ECG module and the precordial electrode array;
Fig. 12A is a top view of the wireless ECG module according to an embodiment of the present disclosure;
Fig. 12B is a side view of wireless ECG module showing the embedded circuitry according to an embodiment of the present disclosure;
Fig. 13 is a diagram of a physiological monitoring system including the precordial electrode array connected to the main module according to an embodiment of the present disclosure; and
Fig. 14 is a side view of the connection between the main module and the precordial electrode array according to an embodiment of the present disclosure.

### Detailed Description

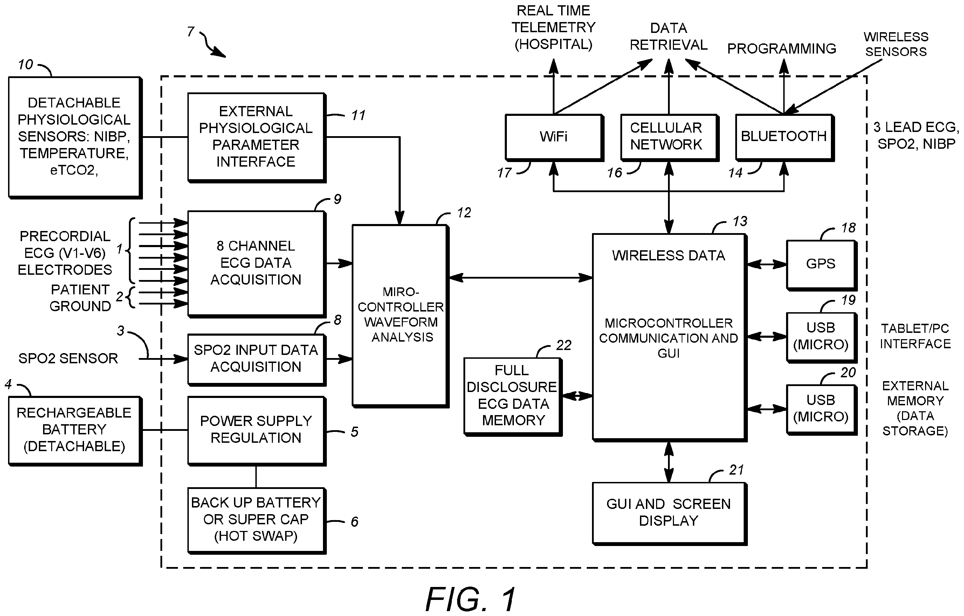

Fig. 1 is a block diagram of a main module for physiological monitoring according to an embodiment of the present disclosure.

As shown in Fig. 1, the main module 7 is attached to several different types of electrodes and sensors known in the art for gathering physiological data related to a patient (e.g., as shown on the left side of Fig. 1). The electrodes and sensors are attached to the main module by, for example, a wired connection. However, the main module 7 can also be connected to wireless sensors using communication interface circuity for receiving data from and sending data to one or more devices using, for example, a Bluetooth connection 14. The data signals from the electrodes and sensors received by the main module 7 include data related to an electrocardiogram (ECG), non-invasive peripheral oxygen saturation (SpO2), non-invasive blood pressure (NIBP), temperature, and/or tidal carbon dioxide (eTCO2). For example, the data signals related to ECG and SpO2 are received respectively from the precordial ECG electrodes 1 and the SpO2 sensor 3. The data signals received from the precordial ECG electrodes 1 and the SpO2 sensor 3 are, for example, analog signals. The data signals from the precordial ECG electrodes 1 are input to the ECG data acquisition circuit 9 and the SpO2 data signal from the SpO2 sensor 3 is input to the SpO2 data acquisition circuit 8. Both the ECG data acquisition circuit 9 and the SpO2 data acquisition circuit 8 include amplifying and filtering circuity as well as analog-to-digital (A/D) circuity that convert the analog signal to a digital signal using amplification, filtering, and A/D conversion methods known in the art.

The data signals related to NIBP, temperature, and eTCO2 are received from a detachable physiological sensors 10 connected to the main module 7 through an external physiological parameter interface 11. The external physiological parameter interface 11 includes, for example, serial interface circuitry for receiving and processing the data signals related to NIBP, temperature, and eTCO2. The processing performed by the ECG data acquisition circuit 9, the SpO2 data acquisition circuit 8, and external physiological parameter interface 11 produces digital data waveforms, which are passed to a dedicated microcontroller 12 by electrical connection therebetween. The digital data waveforms are analyzed by the microcontroller 12 to identify any abnormal conditions of the patient. The microcontroller 12 analyzes the digital waveforms to identify certain digital waveform characteristics and threshold levels indicative of abnormal conditions of the patient using methods known in the art.

The microcontroller 12 is, for example, a processor, a field programmable gate array (FPGA), an application specific integrated circuit (ASIC), a digital signal processor (DSP), or similar processing device. The microcontroller 12 also includes a memory. The memory is, for example, a random access memory (RAM), a memory buffer, a hard drive, a database, an erasable programmable read only memory (EPROM), an electrically erasable programmable read only memory (EEPROM), a read only memory (ROM), a flash memory, or a hard disk.

The memory stores software or algorithms with executable instructions and the microcontroller 12 can execute a set of instructions of the software or algorithms in association with executing an operation of analyzing the digital data waveforms related to the data signals of the electrodes and sensors 1, 3, and 10 to identify abnormal conditions of the patient.

The results of the analysis by the microcontroller 12 are passed to the microcontroller 13 by an electrical connection between the microcontrollers 12, 13. The microcontroller 13 is, for example, a processor, a field programmable gate array (FPGA), an application specific integrated circuit (ASIC), a digital signal processor (DSP), or similar processing device. The microcontroller 13 also includes a memory. The memory is, for example, a random access memory (RAM), a memory buffer, a hard drive, a database, an erasable programmable read only memory (EPROM), an electrically erasable programmable read only memory (EEPROM), a read only memory (ROM), a flash memory, or a hard disk.

Additionally, the microcontroller 13 includes communication interface circuitry for establishing communication connections with various devices and networks using both wired and wireless connections for transmitting physiological data, results of the analysis by the microcontroller 12, and alerts and/or alarms to the patient, clinicians and caregivers regarding any abnormal conditions detected. Additionally, the memory in the microcontroller 13 stores software or algorithms with executable instructions and the microcontroller 13 can execute a set of instructions of the software or algorithms in association with establishing communication connections with various devices and networks using both wired and wireless connections.

As shown in Fig. 1, wireless communication connections established by the communication interface circuity of microcontroller 13 include a Bluetooth connection 14, a cellular network connection 16, and a WiFi connection 17. The wireless communication connections allow for alerts and physiological data to be transmitted in real-time within a hospital wireless communications network (e.g., WiFi) as well as allow for alerts and physiological data to be transmitted in real-time to other devices (e.g., Bluetooth and cellular networks). For example, if the patient monitor (i.e., main module 7) detects a physiological event, an alert or alarm along with pertinent data can transmitted through the cellular network 16 to the clinician and/or health care facility. As another example, if the Bluetooth connection 14 or WIFI connection 17 are not available (e.g., out of transmission range or not operable), and a significant physiological event is detected, the microcontroller 13 can transmit the physiological event, and an alert along with pertinent data using the cellular network connection 16.

It is also contemplated by the disclosure of the present application that the communication connections established by the microcontroller 13 enable communications over other types of wireless networks using alternate hospital wireless communications such as wireless medical telemetry service (WMTS), which can operate at specified frequencies (e.g., 1.4 GHz). Other wireless communication connections can include wireless connections that operate in accordance with, but are not limited to, IEEE802.11 protocol, a Radio Frequency For Consumer Electronics (RF4CE) protocol, ZigBee protocol, and/or IEEE802.15.4 protocol.

The Bluetooth connection 14 can be used to provide the transfer of data to a nearby device (e.g. tablet) for review of data and/or changing of operational settings of the main module 7. The Bluetooth connection 14 also provides wireless communications between the main module 7 and wireless physiological sensors (e.g., ECG, SpO2). Wireless physiological sensors have the advantage of eliminating wires, which get tangled, disconnected, or fail. The microcontroller 13 of the main module 7 provides communication connection by direct wired (e.g., hard-wired) connections as well for transferring data using, for example, a USB connection 19 to a tablet, PC, or similar electronic device; or using, for example, a USB connection 20 to an external storage device or memory.

Additionally, the microcontroller 13 includes a connection to a graphical user interface (GUI) 21 for displaying information, physiological data, measured data, and/or alerts/alarms to the patient, or to clinicians and caregivers proximate to the main module 7. Although the main module 7 is described in Fig. 1 as having two microcontrollers 12, 13, it is contemplated by the disclosure of the present application that one microcontroller could be implemented to perform the functions of the two microcontrollers 12, 13.

The GUI 21 is, for example, a liquid crystal display (LCD), cathode ray tube (CRT), thin film transistor (TFT), light-emitting diode (LED), high definition (HD) or other similar display device with touch screen capabilities. The GUI is provided with means for inputting instructions or information directly to the main module 7.

As shown in Fig. 1, the main module 7 also includes a GPS 18 that can transmit to the clinician or caregiver the location of the patient. If it is determined by the microcontroller 13 that the patient is not within the vicinity of the hospital wireless communications system (e.g., based on input from the GPS 18), the pertinent physiological data (e.g., full disclosure and physiological signal measurements) can be recorded and stored in an on-board memory 22. Additionally, if the Bluetooth connection 14 or WIFI connection 17 are not available (e.g., out of transmission range or not operable), and a physiological event detected is not significant, then the microcontroller can stored the physiological data (e.g., full disclosure and physiological signal measurements) in the on-board memory 22 for later transmission when the Bluetooth connection or WIFI connection become available.

The on-board memory 22 is, for example, a random access memory (RAM), a memory buffer, a hard drive, a database, an erasable programmable read only memory (EPROM), an electrically erasable programmable read only memory (EEPROM), a read only memory (ROM), a flash memory, or a hard disk.

Power can be supplied to the main module 7 using a rechargeable battery 4 that can be detached allowing for replacement. The rechargeable battery 4 is, for example, a rechargeable lithium-ion battery. Additionally, a small built-in back-up battery 6 (or super capacitor) is provided for continuous power to main module 7 during battery replacement. A power supply regulation circuit 5 is provided between the rechargeable battery 4 and small back-up battery 6 to control which of batteries 4, 6 provide power to the main module 7. The main module 7 also includes a patient ground connection 2 for providing a reference when acquiring the ECG signals. The patient ground connection 2 can be used as a ground for single ended unipolar input amplifiers (e.g., precordial leads), or as a ground for bipolar input amplifiers (e.g., limb leads).

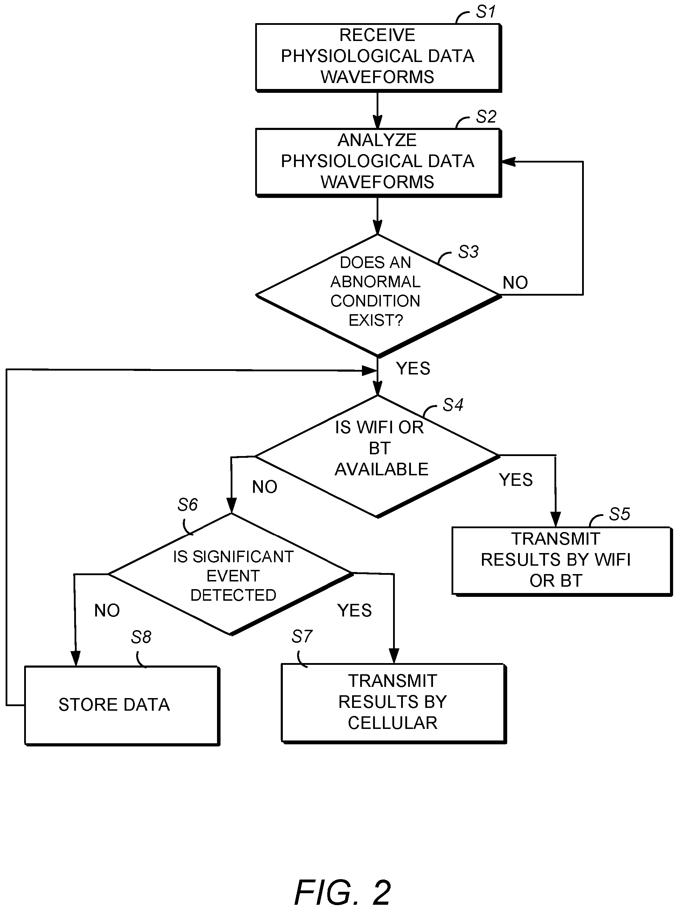

Fig. 2 is an exemplary algorithm executed by the microcontrollers of the main module according to an embodiment of the present disclosure.

In step S1, the microcontroller 12 of the main module 7 receives the digital data waveforms from the ECG data acquisition circuit 9, the SpO2 data acquisition circuit 8, and external physiological parameter interface 11. The memory of the microcontroller 12 has stored in advance digital waveform characteristics and threshold levels indicative of abnormal conditions of the patient. In step S2, the microcontroller 12 analyzes the received digital data waveforms using the stored digital waveform characteristics and threshold levels, and identifies any abnormal conditions by comparing the stored digital waveform characteristics and threshold levels with the received digital data waveforms. In step S3, if it is determined that no abnormal condition exists, the microcontroller 12 continues to analyze the received digital data waveforms that are received, as in step S2. However, in step S3, if it is determined that any abnormal condition exists, then the microcontroller 12 transmits the results of the analysis to the microcontroller 13 of the main module 7 by an electrical connection between the microcontrollers 12, 13.

In step 4, the microcontroller 13 determines if the WIFI connection 17 or the Bluetooth connection 14 is available for transmissions. For example, the microcontroller 13 may determine that the main module 7 is not within transmission range for using the WIFI connection 17 or the Bluetooth connection 14, or determine that the WIFI connection 17 or the Bluetooth connection 14 is not operable. If it is determined by the microcontroller 13 that the WIFI connection 17 or the Bluetooth connection 14 is available, then in step S5 the microcontroller 13 transmits the physiological data and alerts along with other pertinent data using the WIFI connection 17 or the Bluetooth connection 14.

However, in step S4, if it is determined that the WIFI connection 17 or the Bluetooth connection 14 is not available for transmissions, then in step S6 the microcontroller 13 determines if a significant physiological event has been detected (e.g., a significant physiological event requiring immediate attention by a physician or caregiver). If it is determined that a significant physiological event has been detected, then in step S7 the microcontroller 13 transmits the physiological data and alerts along with other pertinent data using the cellular connection 16. However, if it is determined that no significant physiological event has been detected, then in step S8 the microcontroller 13 stores the physiological data in the on-board memory 22 for later transmission when the WIFI connection 17 or the Bluetooth connection 14 become available, as determined in steps S4-S5.

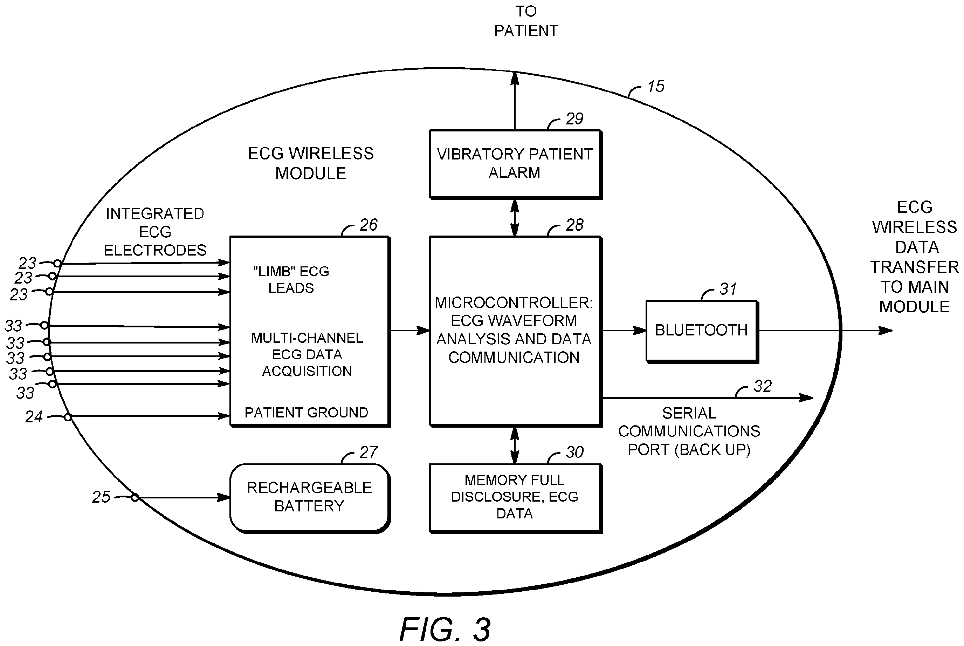

Fig. 3 is a block diagram showing a wireless electrocardiogram (ECG) module according to an embodiment of the present disclosure. The wireless ECG module 15 can have two configurations for physiological data acquisition. During continuous monitoring, the wireless ECG module 15 is connected to a minimal set (e.g., 3) of ECG electrodes 23 that provide data signals related to an electrocardiogram (ECG), similar to 3 channel limb leads known in the art. In an alternative embodiment, additional ECG electrodes can be added. For example, a fourth ECG electrode (not shown) can be added, which can be provided as a ground reference for the data acquisition circuits 26. Additionally, when an acute recording of a 12 lead ECG configuration is required, an additional set of electrodes (e.g., 5 or more) can be added and placed in precordial locations. For example, a precordial array of electrodes can be connected to the wireless ECG module 15 using electrical connections 33 to the data acquisition circuitry 26 (e.g., each electrode having a separate connection 33 to the data acquisition circuitry 26). From the above example, a 12 lead ECG can be derived from the ECG data signals of the 9 electrodes.

As shown in Fig. 3, the ECG electrodes 23 transmit ECG data signals to the data acquisition circuit 26 of the wireless ECG module 15. The ECG electrodes are integrated with the wireless ECG module and transmit ECG data signals by an electrical connection therebetween. The data signals from the ECG electrodes 23 are, for example, analog signals. The data signals from the ECG electrodes 23 are input to an ECG data acquisition circuit 26, which is similar to the data acquisition circuit 9 of the main module 7. That is, the ECG data acquisition circuit 26 includes amplifying circuitry, filtering circuity, and A/D circuity that convert the analog signals to digital signals using amplification, filtering, and A/D conversion methods known in the art.

The processing of the ECG data signals by the ECG data acquisition circuit 26 produces digital data waveforms, which are passed to a microcontroller 28 by electrical connection therebetween. The microcontroller 28 analyzes the digital waveforms to identify certain digital waveform characteristics and threshold levels indicative of abnormal conditions of the patient. The microcontroller 28 is, for example, a processor, a field programmable gate array (FPGA), an application specific integrated circuit (ASIC), a digital signal processor (DSP), or other similar processing device. The microcontroller 28 also includes a memory. The memory is, for example, a random access memory (RAM), a memory buffer, a hard drive, a database, an erasable programmable read only memory (EPROM), an electrically erasable programmable read only memory (EEPROM), a read only memory (ROM), a flash memory, or a hard disk.

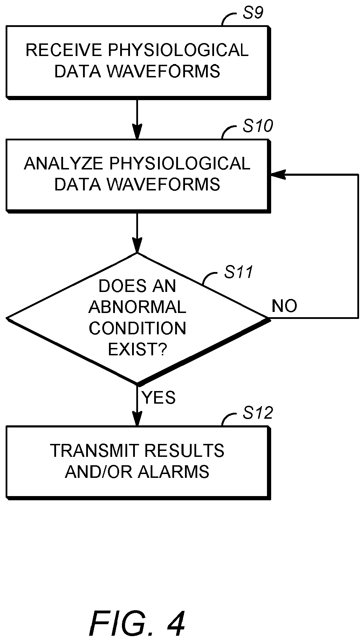

The memory stores software or algorithms with executable instructions and the microcontroller 28 can execute a set of instructions of the software or algorithms in association with executing an operation of analyzing the digital data waveforms related to the data signals of the ECG electrodes 23 to identify abnormal conditions of the patient. Fig. 4 is an exemplary algorithm executed by a microcontroller 28 of the wireless ECG module 15. In step S9, the microcontroller 28 of the wireless ECG module 15 receives the digital data waveforms from the ECG data acquisition circuit 26. The memory of the microcontroller 28 has stored in advance digital waveform characteristics and threshold levels indicative of abnormal conditions of the patient. In step S10, the microcontroller 28 analyzes the received digital data waveforms using the stored digital waveform characteristics and threshold levels, and identifies any abnormal conditions. The microcontroller 28 can identify any abnormal cardiac conditions (e.g. arrhythmias, or ST segment measurements indicative of ischemia or myocardial infarction).

In step S11, if it is determined that no abnormal condition exists, the microcontroller 28 continues to analyze the physiological data waveforms received, as in step S10. However, in step S11, if it is determined that any abnormal condition exists, then in step S12 the microcontroller 28 transmits the results to the patient in the way of an alert or alarm, and/or transmits the results to the main module 7 via a wireless Bluetooth connection 31.

The memory in the microcontroller 28 stores software or algorithms with executable instructions and the microcontroller 28 can execute a set of instructions of the software or algorithms in association with establishing communication connections with various devices and networks using the wireless communication interface circuity of the microcontroller 28.

Referring again to Fig. 3, wireless communication connections established by the wireless communication interface circuity of microcontroller 28 include a Bluetooth connection 31 to the main module 7. The Bluetooth connection 31 enables the microcontroller to transmit alerts and physiological data to the main module 7 in real-time. It is also contemplated by the disclosure of the present application that the communication connections established by the microcontroller 28 enable communications over other types of wireless networks such as wireless connections that operate in accordance with, but is not limited to, IEEE802.11 protocol, a Radio Frequency For Consumer Electronics (RF4CE) protocol, ZigBee protocol, and/or IEEE802.15.4 protocol. As a backup to the wireless connection, alerts and physiological data can be transmitted to the main module 7 in real-time using a serial connection 32.

The microcontroller 28 can also transmit a signal to an internal alarm 29 (i.e., if an abnormal condition is detected) using, for example, a vibratory response to the patient's skin to directly alert the patient. Additionally, pertinent physiological data (e.g., full disclosure and physiological signal measurements) can be stored in on-board memory 30 electrically connected to the microcontroller 28. The on-board memory 30 is, for example, a random access memory (RAM), a memory buffer, a hard drive, a database, an erasable programmable read only memory (EPROM), an electrically erasable programmable read only memory (EEPROM), a read only memory (ROM), a flash memory, or a hard disk. As shown in Fig. 3, power can be supplied to the wireless ECG module 15 by a rechargeable battery 27 that can be recharged via a power connection 25 to the wireless ECG module 15. The rechargeable battery 27 is, for example, a rechargeable lithium-ion battery that can be detached allowing for replacement. The wireless ECG module 15 also includes a patient ground connection 24 for providing as a reference when acquiring the ECG signals. The patient ground connection 24 can be used as a ground for single ended unipolar input amplifiers (e.g., precordial leads), or as a ground for bipolar input amplifiers (e.g., limb leads).

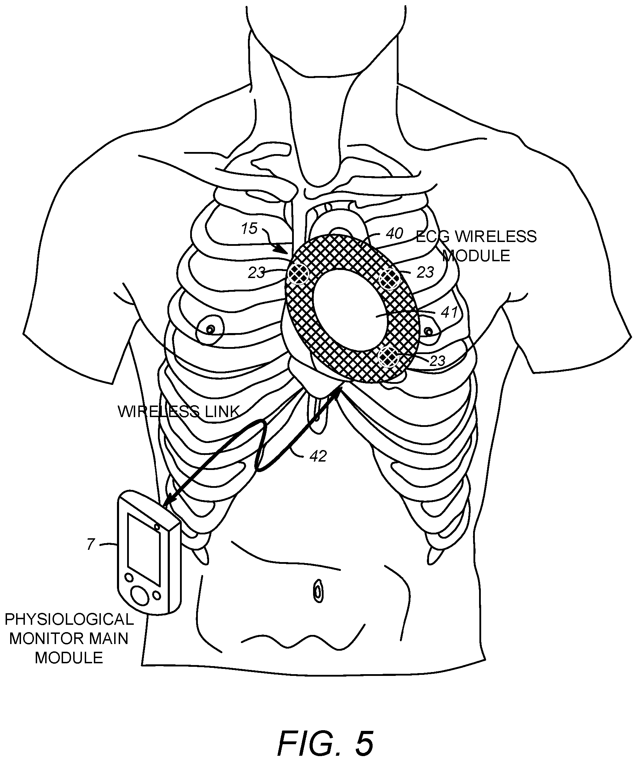

Fig. 5 is a diagram of a physiological monitoring system according to an embodiment of the present disclosure.

As shown in Fig. 5, the physiological monitoring system includes the main module 7 and the wireless ECG module 15 that communicate with each other via a wireless communication link 42. In a preferred embodiment, the wireless communication link 42 established between main module 7 and the wireless ECG module 15 is implemented in accordance with a Bluetooth protocol. The wireless communication link 42 enables the wireless ECG module 15 to transmit alerts and physiological data to the main module 7 in real-time. It is also contemplated by the disclosure of the present application that the wireless communication link 42 established by the wireless ECG module 15 and the main controller 7 is in accordance with other wireless protocols such as, but not limited to, IEEE802.11 protocol, a Radio Frequency For Consumer Electronics (RF4CE) protocol, ZigBee protocol, and/or IEEE802.15.4 protocol. As a backup to the wireless communication link 42, alerts and physiological data is transmitted to the main module 7 in real-time using, for example, a serial connection 32 of the wireless ECG module 15.

As shown in Fig. 5, the main module 7 includes a user interface that provides a means for inputting instructions or information directly to the main module 7. The user interface includes a display screen that is, for example, a liquid crystal display (LCD), cathode ray tube (CRT), thin film transistor (TFT), light-emitting diode (LED), high definition (HD) or other similar display device with touch screen capabilities. The user interface of the main module 7 also provides controls to optimize the viewing of data, which include, but are not limited to, keys, buttons, knobs, touch screen or other similar input devices that can be used to input instructions to the main module 7.

As shown in Fig. 5, the main module 7 is worn by the patient (e.g., on the hip) using a mechanical device or mechanism such as, but not limited to, a clip or strap attached to a surface of the main module 7. However, the main module 7 is detachable and can be removed and held by the patient or set down at a location proximate to the patient. For example, the main module 7 can be placed in a nearby location when the patient is stationary or sleeping (e.g., on a night table).

The wireless ECG module 15 includes a data acquisition module 41 composed of a flexible polymer material with embedded circuity and an electrode patch 40. The embedded circuity of the data acquisition module 41 includes, for example, the data acquisition circuit 26, microcontroller 28, on-board memory 30, the rechargeable battery 27, and patient alarm 29, as described with reference to Fig. 3. The electrode patch 40 of the wireless ECG module 15 is composed of, for example, a breathable porous material integrated with a minimal set (e.g., 3) of ECG electrodes 23. In an alternative embodiment, additional ECG electrodes can be added. Additionally, the electrode patch 40 can also be composed of silicon, polymer, foam, cloth, or similar material.

The electrode patch 40 of the wireless ECG module 15 is attached to the chest of the patient using, for example, a biocompatible adhesive or an adhesive surface on the bottom surface of the electrode patch 40 facing the patient's skin. However, the electrode patch 40 can be made from a material that is self-adhesive. As shown in Fig. 5, the wireless ECG module 15 is located over the heart (e.g., left side of chest) with the electrode patch 40 including the ECG electrodes 23 (e.g., 3) located in a similar orientation as the typical 3 lead (LA, RA, LL) limb lead configuration known in the art. The orientation of the electrodes 23 can also be placed in alternative positions on the patient's chest depending on the patient's anatomy or the area of the heart of diagnostic interest. Once attached to the patient, the electrical signals from the electrodes 23 are received by the data acquisition module 41 using flexible wire interconnections in the wireless ECG module 15 that connect the electrodes to the embedded circuitry of the data acquisition module 41.

The ECG data signals from the electrodes 23 are received by the data acquisition module 41, and processed by the embedded circuity as described previously with reference to Figs. 3 and 4. For example, the data signals from the ECG electrodes 23 are input to an ECG data acquisition circuit 26, which includes amplifying circuitry, filtering circuity, and A/D circuity that convert the analog signals to digital signals using amplification, filtering, and A/D conversion methods known in the art.

The processing of the ECG data signals by the ECG data acquisition circuit 26 produces digital data waveforms that are passed to a microcontroller 28, which analyzes the digital waveforms to identify certain digital waveform characteristics and threshold levels indicative of abnormal conditions of the patient. The microcontroller 28 can identify any abnormal cardiac conditions (e.g. arrhythmias, or ST segment measurements indicative of ischemia or myocardial infarction).

If it is determined that any abnormal condition exists, then the microcontroller 28 transmits the results to the patient in the way of an alert or alarm, and/or transmits the results to the main module 7 via the wireless communication link 42. When transmitting an alert or alarm to the patient, the microcontroller 28 transmits a signal to the internal alarm 29 using, for example, a vibratory response directly to the patient's skin. When the results are transmitted to the main module 7 via the wireless communication link 42, the microcontroller 13 of the main module 7 can establish communication connections using both wired and wireless connections for transmitting physiological data, results, and alerts and/or alarms to the patient, clinicians, and caregivers regarding any abnormal conditions detected as well as store the physiological data in the on-board memory 22, as described previously with reference to Figs. 1 and 2.

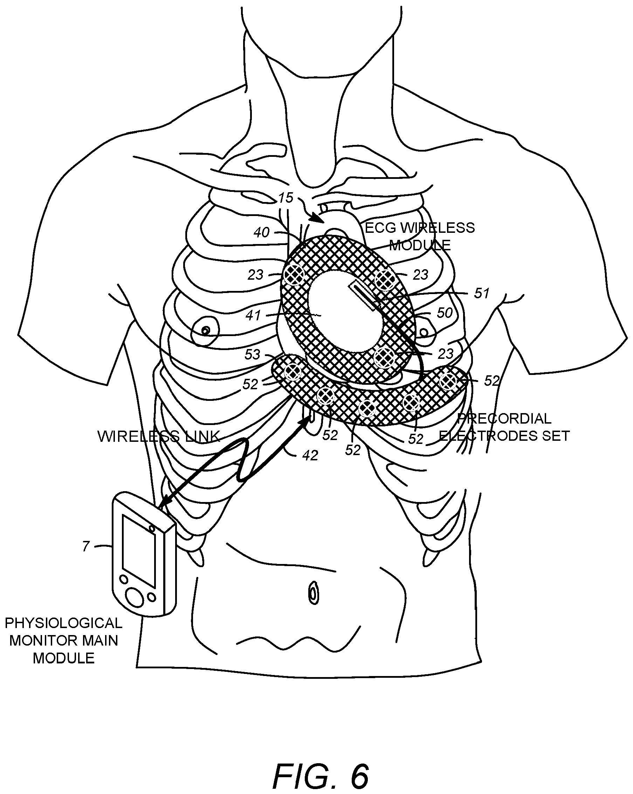

Fig. 6 is a diagram of a physiological monitoring system including a precordial electrode array connected to the wireless ECG module according to an embodiment of the present disclosure.

The physiological monitoring system of Fig. 6 differs from Fig. 5 in that in the physiological monitoring system of Fig. 6 includes a precordial electrode array (or set) 53. The precordial electrode array 53 can be placed over the precordial locations of the right and left chest proximate to the ECG wireless module 15. It is known in the art that the precordial locations are the best locations to detect the heart's electrical activity associated with the septal surface, the anterior wall of the right and left ventricles, and lateral wall of the left ventricle. The precordial electrode array 53 is composed of a breathable porous material integrated with the electrodes 52 (e.g., 5 or more electrodes). However, the precordial electrode array 53 can also be composed of silicon, polymer, foam, cloth, or similar material. The precordial electrode array 53 is attached to the patient using, for example, a biocompatible adhesive or an adhesive surface on the bottom surface of the precordial electrode array 53 facing the patient's skin. However, the precordial electrode array 53 can be made from a material that is self-adhesive.

As shown in Fig. 6, the precordial electrode array 53 is connected to the wireless ECG module 15 through, for example, an external connection port (e.g., ECG cable port) in the data acquisition module 41. The precordial electrode set 53 includes a cable 50 that terminates with an in-line connector 51. The in-line connector 51 is received in the external connection port (e.g., ECG cable port) of the data acquisition module 41, which establishes an electrical connection with the embedded circuity (e.g., connections 33 to the data acquisition circuit 26), thereby also establishing a connection between the precordial electrode array 53 and the wireless ECG module 15. This configuration provides the capability of obtaining additional ECG data for a diagnostic data similar to the 12 lead recording without having to remove the primary electrodes (e.g., 3) of the wireless ECG module 15. A ground reference for the precordial electrode array 53 can be a remote electrode located remote from the precordial set (not shown). Alternatively, the ground reference can be derived from the 3 lead set received through the wireless communication link 42, and converted back to an analog signal. Once the additional 12 lead ECG data is obtained, the precordial electrode array 53 can be disconnected after it is determined that the additional diagnostic ECG data is no longer required. Thus, the 3 lead set does not have to be moved or removed during extended 12 lead ECG recording.

The cable 50 between the precordial electrode set 53 and the wireless ECG module 15 is, for example, an electrical cable or other similar interface cable. Once attached, the electrical signals from the electrodes 52 of the precordial electrode array 53 are received by the data acquisition module 41 of the wireless ECG module 15, and the data signals are processed by the embedded circuity as described previously with reference to Figs. 3 and 4.

For example, the data signals from the electrodes 52 are input to an ECG data acquisition circuit 26, which includes amplifying circuitry, filtering circuity, and A/D circuity that convert the analog signals to digital signals using amplification, filtering, and A/D conversion methods known in the art. The processing of the ECG data signals by the ECG data acquisition circuit 26 produces digital data waveforms that are passed to a microcontroller 28, which analyzes the digital waveforms to identify certain digital waveform characteristics and threshold levels indicative of abnormal conditions of the patient. The microcontroller 28 can identify any abnormal cardiac conditions (e.g. arrhythmias, or ST segment measurements indicative of ischemia or myocardial infarction).

If it is determined that any abnormal condition exists, then the microcontroller 28 transmits the results to the patient in the way of an alert or alarm, and/or transmits the results to the main module 7 via the wireless communication link 42. When transmitting an alert or alarm to the patient, the microcontroller 28 transmits a signal to the internal alarm 29 using, for example, a vibratory response directly to the patient's skin. When the results are transmitted to the main module 7 via the wireless communication link 42, the microcontroller 13 of the main module 7 can establish communication connections using both wired and wireless connections for transmitting physiological data, results, and alerts and/or alarms to the patient, clinicians, and caregivers regarding any abnormal conditions detected as well as store the physiological data in the on-board memory 22, as described previously with reference to Figs. 1 and 2.

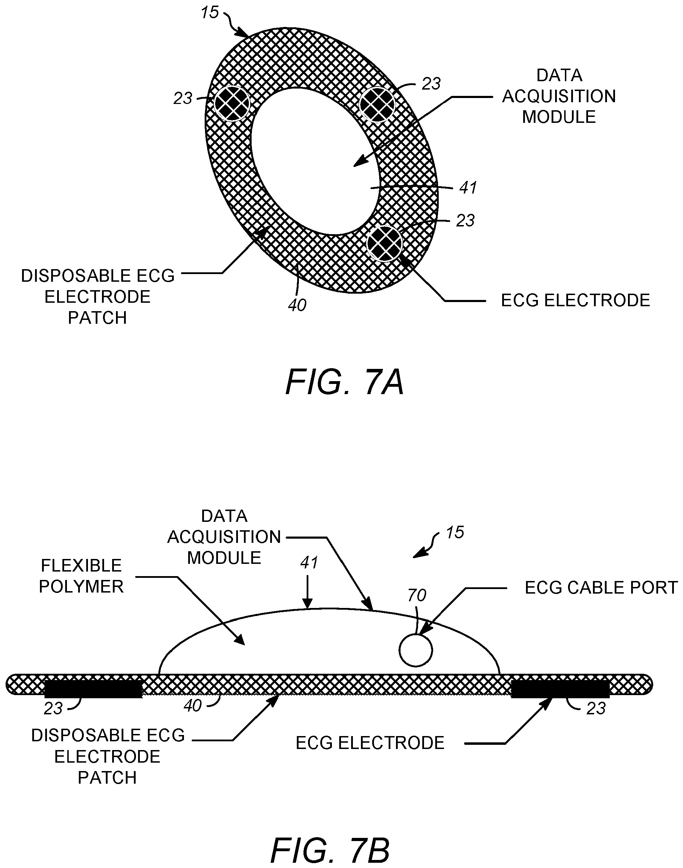

Fig. 7A is a top view of the wireless ECG module and Fig. 7B is a side view of the wireless ECG module according to an embodiment of the present disclosure.

As shown in Fig. 7A, the wireless ECG module 15 includes a data acquisition module 41 with embedded circuity and an electrode patch 40. The embedded circuity of the data acquisition module 41 includes, for example, the data acquisition circuit 26, microcontroller 28, on-board memory 30, the rechargeable battery 27, and patient alarm 29, as described with reference to Fig. 3. The data acquisition module 41 is composed of, for example, a flexible polymer or other similar material.

The electrode patch 40 of the wireless ECG module 15 is composed of, a breathable porous material integrated with a minimal set (e.g., 3) of ECG electrodes 23, similar to 3 channel limb leads known in the art. However, the electrode patch can also be composed of silicon, polymer, foam, cloth, or similar material.

In this embodiment, the ECG data acquisition module 41 can be a reusable device and detachably connected to the electrode patch 40, whereas the electrode patch 40 (including the electrodes) is disposable. The ECG electrode patch 40 is composed of a material that is a flexible porous structure to allow the patient's skin to "breathe." The ECG data acquisition module 41 is composed of a flexible material such as a polymer that is resistant to water ingress, which allows the patient to take a shower or bath while wearing the wireless ECG module 15. The polymer can also be flexible to allow the wireless ECG module 15 to conform to the patient's body. Once the ECG data acquisition module 41 is attached to a new electrode patch 40, the three (3) electrodes are connected underneath the ECG electrode patch by flexible circuity to the data acquisition module 41 for detecting the ECG voltage signals.

Fig. 7B illustrates a side view of the wireless ECG wireless module 15 with the ECG data acquisition module 41 attached to the ECG electrode patch 40. As shown in Fig. 7B, the electrodes 23 are located on the outer periphery of the electrode patch 40 with a bottom surface of the electrodes configured to come into contact with the patient's skin. In this embodiment, the ECG data acquisition module 41 is a reusable device and detachably connected to the electrode patch 40, whereas the electrode patch 40 (including the electrodes) is disposable and composed of a material that is a flexible porous structure to allow the patient's skin to "breathe." The ECG data acquisition module 41 is composed of a flexible material (e.g., silicone or polymer) that provides protection for the electrical interconnects between the data acquisition module 41 and the electrode patch 40 against environmental hazards such as moisture.

When the patient is within a higher acuity level of physiological monitoring, there can still be a requirement to obtain a 12 lead ECG recording at certain times throughout the course of a day for diagnostic purposes. It is also preferable that the clinicians do not have to remove the electrodes for the 3 lead configuration, to attach electrodes for a separate 12 lead ECG recorder. Under such conditions, a separate precordial ECG electrode array 53 can be attached directly to the patient and the wireless ECG module 15 using the cable port 70 in the data acquisition module 41 for providing additional ECG recordings in precordial locations during the higher acuity monitoring. The precordial electrode set 53 includes a cable 50 that terminates with an in-line connector 51. The in-line connector 51 is received in the ECG cable port 70 of the data acquisition module 41, which establishes an electrical connection with the embedded circuity (e.g., connections 33 to the data acquisition circuit 26), thereby also establishing a connection between the precordial electrode array 53 and the wireless ECG module 15.

The ECG electrode array 53 can also be used during cardiac rehabilitation activities to monitor the 12 lead ECG signal. Once the requirement for obtaining the precordial ECG waveforms is no longer required, the precordial ECG electrode array 53 can be disconnected from the wireless ECG module 15 by disconnecting the in-line connector 51 of the cable 50 from the ECG cable port 70 of the wireless ECG module. The precordial electrode array 53 can be removed from the patient leaving the primary 3 lead ECG patch 40 of the wireless ECG module 15 attached for additional continuous 24 hour (or greater) monitoring.

In another embodiment of the ECG electrode patch 40 of the ECG wireless module 15, the ECG electrode patch 40 can include additional electrodes (e.g. 5 or more) embedded within a flexible polymer insulation for detecting the ECG voltage signal as a precordial ECG electrode array.

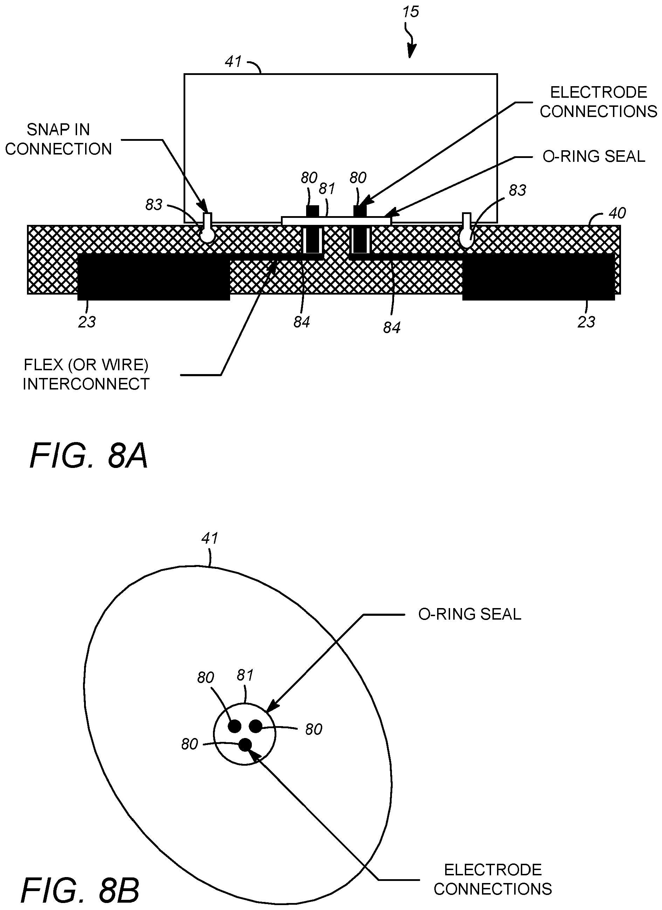

Fig. 8A is side view of the connection between the data acquisition module and the electrode patch of the ECG module according to an embodiment of the present disclosure.

As shown in Fig. 8A, the connections between the data acquisition module 41 and the electrode patch 40 includes mechanical connections 83, electrical connections 80, 84, and an O-ring seal 81 to protect against water ingress, which allows the patient to shower or take a bath while wearing the wireless ECG module 15. The mechanical connections are, for example, snap plug mechanisms 83 that snap into the surface of the electrode patch 40, thereby detachably securing the bottom surface of the data acquisition module 41 to the top surface of the electrode patch 40. The electrical connections include, for example, male electrical connectors 80 (e.g., 3) that establish an electrical connection with the electrodes 23 of the electrode patch 40 by coming in contact with the wires or flexible circuit 84 embedded in the electrode patch 40. The electrical connections 80, 84 allow for ECG signals from the ECG electrodes 23 to be transmitted to the ECG data acquisition module 41. Although Fig. 8A shows the use of male connectors 80, it is also contemplated by the disclosure of the present application that the connectors used for establishing connections between the data acquisition module 41 and the electrode patch 40 are female connectors or a combination of male and female connectors.

In order to protect against water ingress to the electrical connection between the data acquisition module 41 and the electrode patch 40, there is an O-ring seal 81 around the electrical connectors 80 and positioned between the bottom surface of the data acquisition module 41 and the top surface of the ECG electrode patch 40.

Fig. 8B is a bottom view of the electrode connections of the data acquisition module according to an embodiment of the present disclosure.

As shown in Fig. 8B, the bottom surface of the data acquisition module 41 includes an O-ring seal 81 that is concentric and surrounding the electrical connectors 80 (e.g., 3 connectors) in order to provide a water tight seal, which protects the integrity of the electrical connection between the data acquisition module 41 and the electrode patch 40. The water tight seal created by the O-ring seal 81 also allows the patient to take a shower or bath while wearing the wireless ECG module 15.

The electrical connectors 80 are, for example, male electrical connectors (e.g., 3) that establish an electrical connection with the electrodes 23 of the electrode patch 40 coming in contact with the wires or flexible circuit 84 embedded in the electrode patch 40. It is contemplated by the disclosure of the present application that the connectors used for establishing connections between the data acquisition module 41 and the electrode patch 40 can be male or female connectors or a combination of male and female connectors.

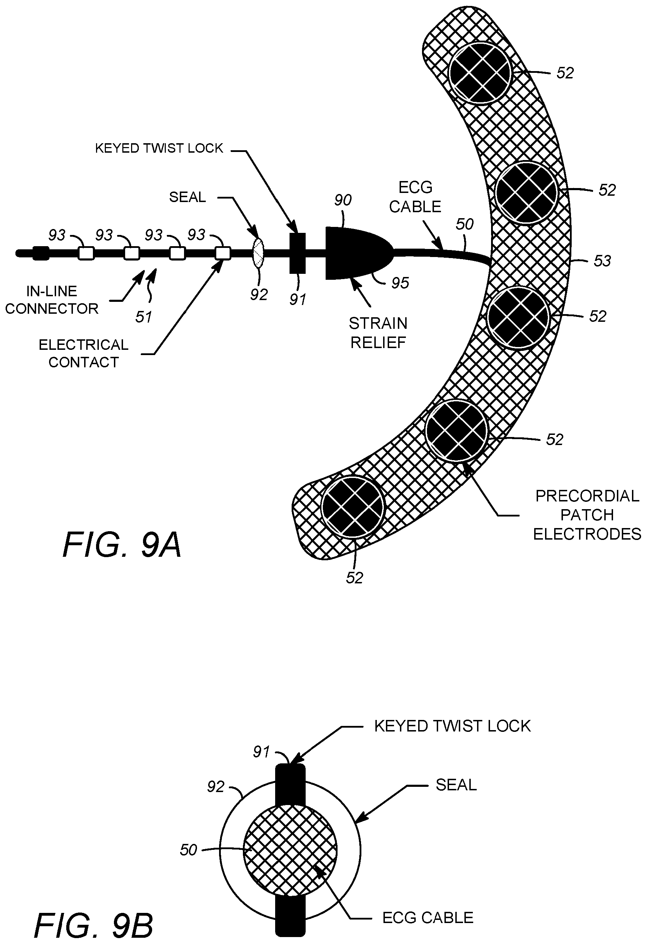

Fig. 9A is top view of a precordial electrode array with cable and in-line connector according to an embodiment of the present disclosure.

A separate precordial ECG electrode array 53 can be attached directly to the patient and the wireless ECG module 15 using the cable port 70 in the data acquisition module 41 for providing additional ECG recordings in precordial locations during the higher acuity monitoring. The precordial electrode array 53 is attached to the chest of the patient using, for example, a biocompatible adhesive or an adhesive surface on the bottom surface of the precordial electrode array 53 facing the patient's skin. However, the precordial electrode array 53 can be made from a material that is self-adhesive. The precordial electrode array 53 is connected to the primary set of electrodes (e.g., 3 electrodes) of the wireless ECG module 15 through an external connection port (e.g., ECG cable port) in the data acquisition module 41.

The precordial electrode array 53 is composed of a breathable porous material integrated with the electrodes 52 (e.g., 5 or more electrodes). However, the precordial electrode array 53 can also be composed of silicon, polymer, foam, cloth, or similar material. As shown in Fig. 9A, the precordial electrode array 53 includes a cable 50 that terminates with an in-line connector 51. The in-line connector 51 includes a series of electrical contacts 93 that are received in the external connection port (e.g., ECG cable port 70) of the data acquisition module 41 and establish an electrical connection with the embedded circuity (e.g., connections 33 to the data acquisition circuit 26) and thereby a connection between the precordial electrode array 53 and the wireless ECG module 15. This configuration provides the capability of obtaining additional ECG data as diagnostic data similar to the 12 lead recording without having to remove the primary electrodes (e.g., 3) of the wireless ECG module 15.

The in-line connector 51 also includes a seal 92 that is concentric around the ECG cable 50 for providing a water tight seal, thereby protecting the integrity of the electrical connection between the data acquisition module 41 and the precordial electrode array 53 when the in-line connector is received in the external connection port (e.g., ECG cable port 70) of the data acquisition module 41. Having the seal 91 integrated within the ECG cable 50 rather than having an O-ring within the connector cavity can improve the reliability of the seal by avoiding a multi-use configuration.

The in-line connector 51, once establishing a connection between the data acquisition module 41 and the precordial electrode array 53, is held securely in place using a lock mechanism such as a keyed twist lock 91. When the keyed twist lock 91 is inserted in the external connection port of the data acquisition module 41, the keyed twist lock 91 is twisted (e.g., 90° in the clockwise direction) and aligned within a grooved slot (e.g., in the external connection port). The In-line connector 51 is guided into place in the external connection port of the data acquisition module 41 by holding the strain relief portion 90 of the in-line connector 51, which prevents damage to the precordial electrode array 53 and the cable 50. The strain relief portion 90 is integrated into the ECG cable 50, which can also provide additional protection from water ingress.

The ECG electrode array 53 can be used during cardiac rehabilitation activities to monitor the 12 lead ECG signal. Once the requirement for obtaining the precordial ECG waveforms is no longer required, the precordial ECG electrode array 53 can be disconnected from the wireless ECG module 15 by disconnecting the in-line connector 51 of the cable 50 from the ECG cable port 70 of the wireless ECG module 15. The precordial electrode array 53 can be removed from the patient leaving the primary 3 lead ECG patch 40 of the wireless ECG module 15 attached for additional continuous 24 hour (or greater) monitoring.

Fig. 9B is a cross-sectional view of the keyed twist lock of the ECG cable for the precordial electrode array according to an embodiment of the present disclosure.

The in-line connector 51, once in place and establishing a connection between the data acquisition module 41 and the precordial electrode array 53, is held securely in place using an integrated keyed twist lock 91.

As shown in Fig. 9B, the keyed twist lock 91 is integrated with the cable 50, similar to the seal 92. The key twist lock 91 is inserted into a grooved slot of the external connection port (e.g., ECG cable port 70) of the data acquisition module 41 and twisted, for example, 90° in a clockwise direction and aligned within a grooved slot, thereby securing the connection between the in-line connector 51 and the data acquisition module 41. Once the requirement for obtaining the precordial ECG waveforms is no longer required, the precordial ECG electrode array 53 can be disconnected from the wireless ECG module 15 by again twisting the key twist lock 91 inserted into a grooved slot of the external connection port by, for example, 90° in a counter clockwise direction, thereby releasing the connection between the in-line connector 51 and the data acquisition module 41.

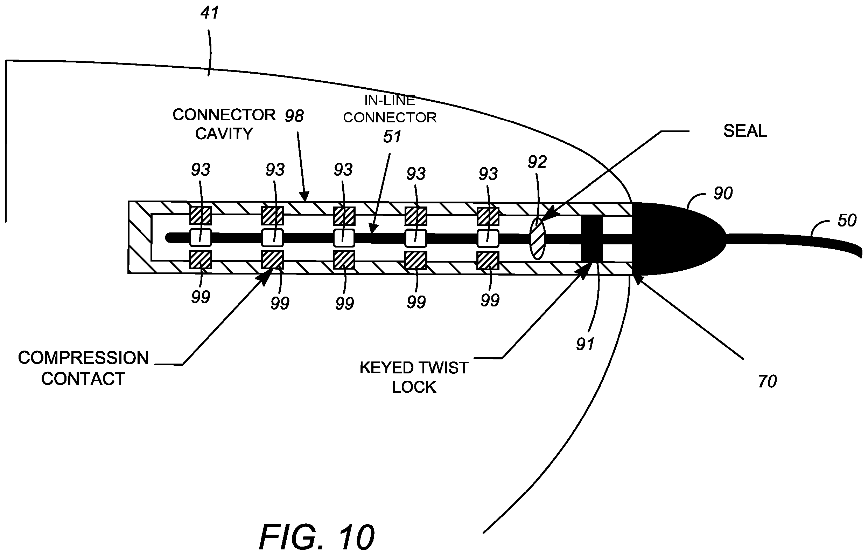

Fig. 10 is a side view of the connection between the data acquisition module and the precordial electrode array according to an embodiment of the present disclosure.

The precordial electrode array 53 includes a cable 50 that terminates with an in-line connector 51. As shown in Fig. 10, the in-line connector 51 includes a series of electrical contacts 93 that are received in the ECG cable port 70 of the data acquisition module 41. The ECG cable port 70 includes a connector cavity 98 having a series of compression contacts 99 that align with the series of electrical contacts 93 when the in-line connector 51 is fully inserted into the connector cavity 98 of the data acquisition module 41. When the in-line connector 51 is fully inserted into the connector cavity 98 of the data acquisition module 41, the in-line connector 51 establishes an electrical connection with the embedded circuity (e.g., connections 33 to the data acquisition circuit 26) of the data acquisition module 41, which in turn establishes a connection between the precordial electrode array 53 and the wireless ECG module 15. This configuration provides the capability of obtaining additional ECG data for diagnostic data similar to the 12 lead recording without having to remove the primary electrodes (e.g., 3) of the wireless ECG module 15.

The in-line connector 51 also includes an integrated seal 92 that is concentric around the ECG cable 50 for providing a water tight seal, thereby protecting the integrity of the electrical connection between the data acquisition module 41 and the precordial electrode array 53 when the in-line connector 51 is received in the ECG cable port 70 of the data acquisition module 41. Having the seal 92 integrated within the ECG cable 50 rather than having an O-ring within the connector cavity can improve the reliability of the seal by avoiding a multi-use configuration.

The in-line connector 51, once establishing a connection between the data acquisition module 41 and the precordial electrode array 53, is held securely in place using a lock mechanism such as a keyed twist lock 91, which when inserted in the ECG cable port 70 of the data acquisition module 41 is twisted (e.g., 90°) and aligned within a grooved slot. The In-line connector 51 is guided into ECG cable port 70 and the connector cavity 98 by holding the strain relief portion 90 of the in-line connector 51, which prevents damage to the precordial electrode array 53 and the cable 50. The strain relief portion 90 is integrated into the ECG cable 50, which can also provide additional protection from water ingress.

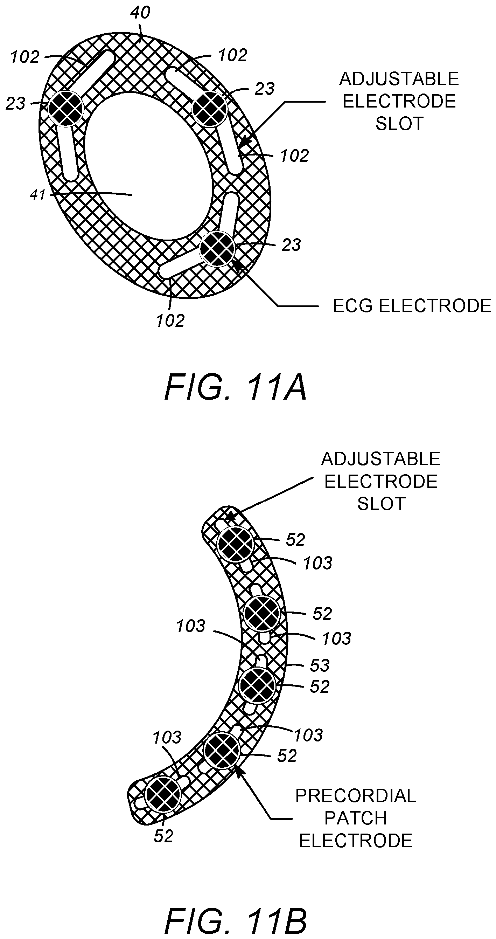

Figs. 11A and 11B are top views respectively of the adjustable electrodes on the ECG module and the precordial array.

Each patient has a different anatomy (e.g., body size and orientation of heart) and may require monitoring for different cardiac conditions. Therefore, there may be a requirement to adjust the location of each ECG electrode to be application specific for each patient. As shown in FIG. 11A, this embodiment of the wireless ECG module 15 includes a data acquisition module 41 composed of a flexible polymer material with embedded circuity and an electrode patch 40. The embedded circuity of the data acquisition module 41 includes, for example, the data acquisition circuit 26, microcontroller 28, on-board memory 30, the rechargeable battery 27, and patient alarm 29, as described with reference to Fig. 3.

The electrode patch 40 of the wireless ECG module 15 is composed of, for example, a breathable porous material integrated with a minimal set (e.g., 3) of ECG electrodes 23. In an alternative embodiment, additional ECG electrodes can be added. Additionally, the electrode patch 40 can also be composed of silicon, polymer, foam, cloth, or similar material. As shown in Fig. 11A, the electrode patch 40 includes adjustable electrodes slots 102. The location of each ECG electrodes 23 can be adjusted within each of electrode slots 102 in the electrode patch 40. For example, one potential method to adjust each ECG electrode 23 would be to provide an adjustment to each ECG electrode 23 within its corresponding adjustable electrode slot 102 and relying on the compression of the flexible polymer of the electrode patch 40 around the body of the ECG electrodes 23 or an electrode adhesive to maintain stability.

As shown in FIG. 11B, this embodiment of the precordial electrode array 53 includes adjustable electrode slots 103. The precordial electrode array 53 is composed of a breathable porous material integrated with the electrodes 52 (e.g., 5 or more electrodes). However, the precordial electrode array 53 can also be composed of silicon, polymer, foam, cloth, or similar material. The location of each ECG electrode 52 can be adjusted within its corresponding electrode slot 103 in the precordial electrode array 53. For example, each ECG electrode 52 can be adjusted within its corresponding adjustable electrode slot 103 by relying on the compression of the flexible polymer of the around the body of the ECG electrodes 53 or an electrode adhesive to maintain stability.

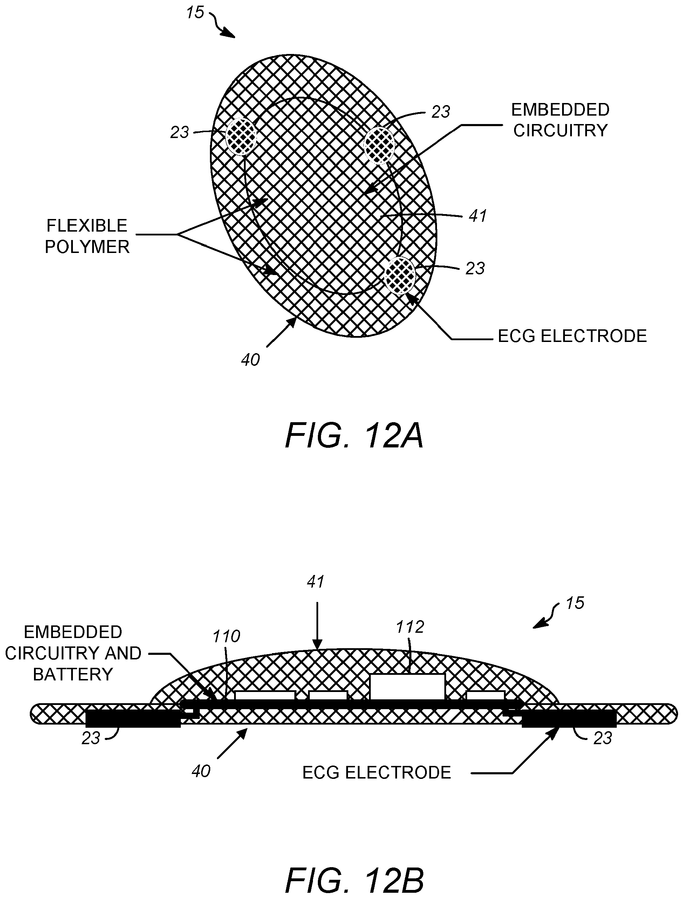

Figs. 12A and 12B show an embodiment of the present disclosure in which the ECG data acquisition module 41 is integrated with the electrode patch 40 such that the entire ECG wireless device 15 is disposable. The wireless ECG module 15 is composed of, for example, a breathable porous material integrated with a minimal set (e.g., 3) of ECG electrodes 23. In an alternative embodiment, additional ECG electrodes can be added. Additionally, the wireless ECG module 15 also be composed of silicon, polymer, foam, cloth, or similar material.

As shown in Fig. 12B, the electrodes 23 are located on the outer periphery of the electrode patch 40 with a bottom surface of the electrodes configured to come into contact with the patient's skin. In this embodiment, the ECG data acquisition module 41 and the electrode patch 40 are disposable, and the data acquisition module 41 of wireless ECG module 15 includes embedded circuity 112. The embedded circuity includes, for example, the data acquisition circuit 26, microcontroller 28, on-board memory 30, the rechargeable battery 27, and patient alarm 29, as described with reference to Fig. 3.

As shown in Fig. 12B, electrical connection is establish between electrodes 23 of the electrode patch 40 and the embedded circuity 112 by wires or a flexible circuit 110 embedded in the wireless ECG module 15. The electrical connection allows for ECG signals from the ECG electrodes 23 to be transmitted to the embedded circuit 110 of the ECG data acquisition module 41.

The embodiment of Figs. 12A and 12B of the wireless ECG module 15 can simplify the interconnection design and would eliminate the requirement for a rechargeable power source (i.e. having to swap out between two ECG data acquisition modules). From a clinical perspective, a totally disposable device can improve the patient's outcome by reducing the risk of infection which at times may be caused from improper cleaning methods associated with reusable devices.

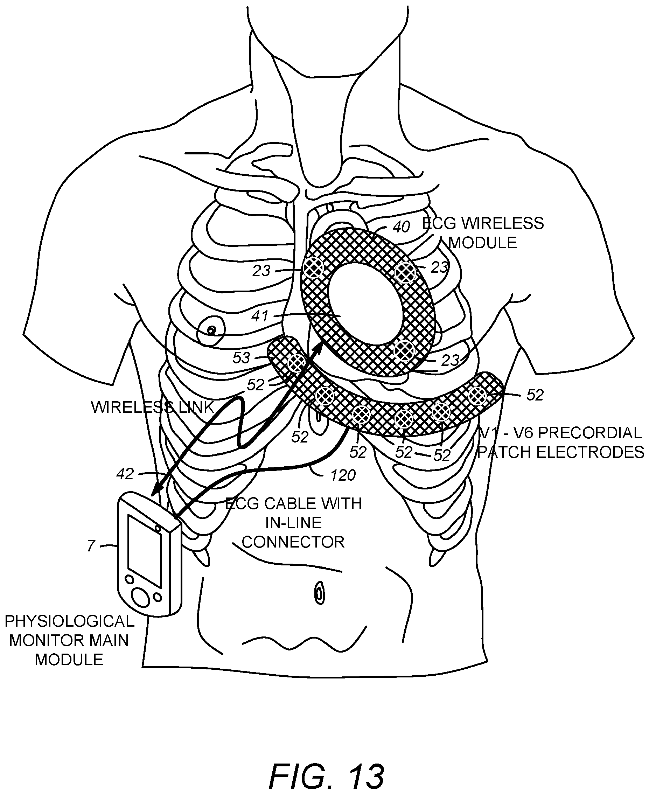

Fig. 13 is a diagram of a physiological monitoring system including the precordial electrode array connected to the main module according to an embodiment of the present disclosure.

The physiological monitoring system of Fig. 13 differs from the physiological monitoring system of Fig. 6 in that the precordial electrode array (or set) 53 is connected to the main module 7 instead of being connected to the wireless ECG module 15. The precordial electrode array 53 (e.g., 5 or more electrodes) can be placed over the precordial locations of the right and left chest proximate to the ECG wireless module 15. The precordial electrode array 53 is composed of a breathable porous material integrated with the electrodes 52. However, the precordial electrode array 53 can also be composed of silicon, polymer, foam, cloth, or similar material.

The precordial electrode array 53 is attached to the patient using, for example, a biocompatible adhesive or an adhesive surface on the bottom surface of the precordial electrode array 53 facing the patient's skin. However, the precordial electrode array 53 can be made from a material that is self-adhesive.

The precordial electrode array 53 is connected to the main module 7 by a cable 120 inserted into an external connection port (e.g., cable port) in the main module 7. The precordial electrode set 53 includes a cable 120 that terminates with an in-line connector. The in-line connector is received in the external connection port of the main module 7, thereby establishing a connection between the precordial electrode array 53 and the circuity of the main module 7. This configuration provides the capability of obtaining additional ECG data for a diagnostic data similar to the 12 lead recording without having to remove the primary electrodes (e.g., 3) of the wireless ECG module 15. Once the additional 12 lead ECG data is obtained, the precordial electrode array 53 can be disconnected after it is determined that the additional diagnostic ECG data is no longer required.

The cable 120 between the precordial electrode set 53 and the main module 7 is, for example, an electrical cable or other similar interface cable. Once attached, the electrical signals from the electrodes 52 of the precordial electrode array 53 are received by the main module 7, the data signals are processed by the circuity of the main module 7 as described previously with reference to Figs. 1 and 2.

For example, the data signals from the electrodes 52 are input to an ECG data acquisition circuit 9, which includes amplifying circuitry, filtering circuity, and A/D circuity that convert the analog signals to digital signals using amplification, filtering, and A/D conversion methods known in the art. The processing of the ECG data signals by the ECG data acquisition circuit 9 produces digital data waveforms, which are passed to a microcontroller 12, which analyzes the digital waveforms to identify certain digital waveform characteristics and threshold levels indicative of abnormal conditions of the patient. The microcontroller 12 can identify any abnormal cardiac conditions (e.g. arrhythmias, or ST segment measurements indicative of ischemia or myocardial infarction).

Additionally, the microcontroller 13 includes communication interface circuitry for establishing communication connections with various devices and networks using both wired and wireless connections for transmitting physiological data, results of the analysis by the microcontroller 12, and alerts and/or alarms to the patient, clinicians and caregivers regarding any abnormal conditions detected as well as storing the physiological data in the on-board memory 22.

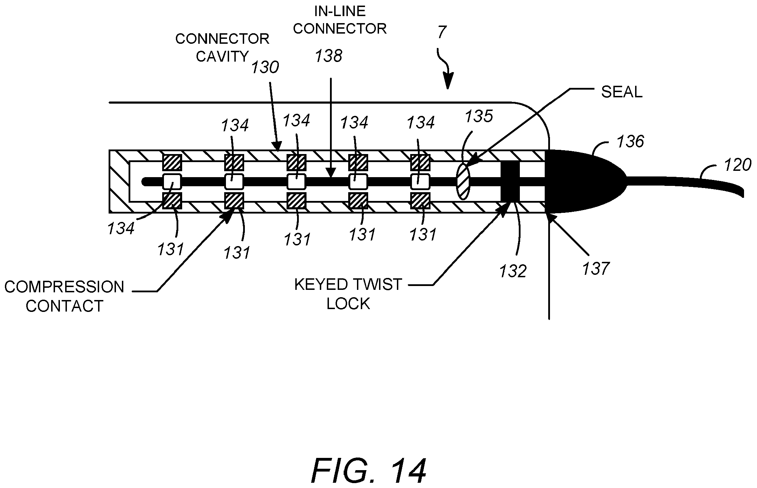

Fig. 14 is a side view of the connection between the main module and the precordial electrode array according to an embodiment of the present disclosure.

The precordial electrode array 53 includes a cable 120 that terminates with an in-line connector 138. As shown in Fig. 14, the in-line connector 138 includes a series of electrical contacts 134 that are received in the cable port 137 of the main module 7. The cable port 137 includes a connector cavity 130 having a series of compression contacts 131 that align with the series of electrical contacts 134 when the in-line connector 138 is fully inserted into the connector cavity 130 of the main module 7. When the in-line connector 138 is fully inserted into the connector cavity 130 of the main module 7, the in-line connector 138 establishes an electrical connection with the circuity of the main module 7.

The in-line connector 138 also includes an integrated seal 135 that is, for example, concentric around the ECG cable 120 for providing a water tight seal, thereby protecting the integrity of the electrical connection between the and the precordial electrode array 53 and the main module 7 when the in-line connector 138 is received in the cable port 137. Having the seal 135 integrated within the ECG cable 120 rather than having an O-ring within the connector cavity 130 can improve the reliability of the seal 135 by avoiding a multi-use configuration.

The in-line connector 138, once establishing a connection between the main module 7 and the precordial electrode array 53, is held securely in place using a lock mechanism such as a keyed twist lock 132. The keyed twist lock 132 is inserted in the ECG cable port 137 of the main module 7 and then twisted (e.g., 90°) and aligned within a grooved slot (e.g., in the connector cavity). The In-line connector 138 is guided into cable port 137 and the connector cavity 130 (i.e., for establishing a connection between the main module 7 and the precordial electrode array 53 by holding a strain relief portion 136 of the in-line connector 138, which prevents damage to the precordial electrode array 53 and the cable 120. The strain relief portion 136 is integrated into the ECG cable 120, which can also provide additional protection from water ingress.

The subject matter described in the present disclosure of the present application provides many technical improvements over conventional patient monitoring devices and systems that includes, for example, improved outcomes of patients during ambulatory activity associated with their recuperation and rehabilitation by providing a simplified ECG electrode placement, a detachable precordial electrode array, wireless communications between product subsystems which eliminates wires between ECG electrodes and physiological monitor, and improved performance with respect to noise immunity and reliability.

Technical improvements over conventional patient monitoring devices and systems also include interconnections of the electrode array to the main module and the wireless ECG electrode array, which can be configured with an in-line connection. Additionally, the electrode array and the wireless ECG module have the capability to adjust individual electrode locations which could allow for application specific cardiac vectors to each patient. Moreover, the wireless ECG module can include disposable components and circuitry.

The present disclosure may be implemented as any combination of an apparatus, a system, an integrated circuit, and a computer program on a non-transitory computer readable recording medium. The microcontrollers may be implemented as an integrated circuit (IC), an application specific integrated circuit (ASIC), or large scale integrated circuit (LSI), system LSI, super LSI, or ultra LSI components which perform a part or all of the functions of the wireless ECG module and main module.

Each of the components of the wireless ECG module and the main module of the present disclosure can be implemented using many single-function components, or can be one component integrated using the technologies described above. The various illustrative circuits and modules described in connection with the disclosure herein may be implemented or performed with a general-purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices, e.g., a combination of a DSP and a microprocessor, multiple microprocessors, one or more microprocessors in conjunction with a DSP core, or any other such configuration. A processor may in some cases be in electronic communication with a memory, where the memory stores instructions that are executable by the processor.

The present disclosure includes the use of computer programs or algorithms in the wireless ECG module and the main module. The programs or algorithms can be stored on a non-transitory computer-readable medium for causing a computer, such as the microcontroller, to execute the steps described in Figs. 2 and 4. The computer programs, which can also be referred to as programs, software, software applications, applications, components, or code, include machine instructions for a programmable processor, and can be implemented in a high-level procedural language, an object-oriented programming language, a functional programming language, a logical programming language, or an assembly language or machine language. The term computer-readable recording medium refers to any computer program product, apparatus or device, such as a magnetic disk, optical disk, solid-state storage device, memory, and programmable logic devices (PLDs), used to provide machine instructions or data to a programmable data processor, including a computer-readable recording medium that receives machine instructions as a computer-readable signal.

By way of example, computer-readable medium can comprise RAM, ROM, EEPROM, CD-ROM or other optical disk storage, magnetic disk storage or other magnetic storage devices, or any other medium that can be used to carry or store desired computer-readable program code in the form of instructions or data structures and that can be accessed by a general-purpose or special-purpose computer, or a general-purpose or special-purpose processor. Disk or disc, as used herein, include compact disc (CD), laser disc, optical disc, digital versatile disc (DVD), floppy disk and Blu-ray disc where disks usually reproduce data magnetically, while discs reproduce data optically with lasers. Combinations of the above are also included within the scope of computer-readable media.

The subject matter of the disclosure of the present application are merely provided as examples of patient monitoring devices and systems. Further features or variations are contemplated in addition to the features of the patient monitoring apparatus and systems described above. It is contemplated that the implementation of the components of the present disclosure can be done with any newly arising technology that may replace any of the above implemented technologies.

The above description provides examples, and is not limiting of the scope, applicability, or configuration set forth in the claims. Changes may be made in the function and arrangement of elements discussed. Various embodiments may omit, substitute, or add various procedures or components as appropriate. For instance, features described with respect to certain embodiments may be combined in other embodiments.

The previous description of the disclosure is provided to enable a person skilled in the art to make or use the disclosure. Various modifications to the disclosure will be readily apparent to those skilled in the art, and the generic principles defined herein may be applied to other variations. Throughout this disclosure the term "example" indicates an example or instance and does not imply or require any preference for the noted example.

## Claims

1. A physiological monitoring system for providing monitoring of a patient, comprising
an electrocardiogram (ECG) (15) module including an ECG microcontroller (28) and a first plurality of electrodes (23) worn on the patient, the ECG microcontroller (28) being coupled to the first plurality of electrodes (23) for receiving first physiological data gathered by the first plurality of electrodes (23);
a main module (7) detachably worn by the patient and connected to the ECG module by a wireless communication connection (42), the main module including a main controller, an on-board memory (22, 30), and a communication interface coupled to the main controller,
**characterized by**:
the communication interface including a first wireless communication protocol and a second wireless communication protocol different from the first wireless communication protocol;
the ECG microcontroller (28) being configured to:
analyze the first physiological data,
identify a first abnormal condition of the patient based on the analyzed first physiological data,
determine a first significant physiological event based on the identified first abnormal condition of the patient,
transmit to the main controller (7) in real-time, using the wireless communication connection, data corresponding to the first significant physiological event;
the main controller being configured to:
receive the data corresponding to the first significant physiological event from the ECG module using the wireless communication connection (42);
transmit in real-time, using the first wireless communication protocol, data corresponding to the first significant physiological event, and
when the first wireless communication protocol is inoperable, transmit in real-time, using the second wireless communication protocol, the data corresponding to the first significant physiological event.

2. The physiological monitoring system of claim 1, wherein the first wireless communication protocolis in accordance with WIFI (17) or Bluetooth (14).

3. The physiological monitoring system as in any one of the preceding claims, wherein the second wireless communication protocol is in accordance with a cellular network.

4. The physiological monitoring system of any preceding claim, further comprising a detachable precordial electrode array (53) including a second plurality of electrodes (52) worn in a precordial location of the patient proximate to the ECG module, wherein:
the second plurality of electrodes (52) being configured to gather second physiological data,
the detachable precordial electrode array (53) is configured to transmit the second physiological data to the ECG module by a wired or wireless communication connection.

5. The physiological monitoring system of claim 4, wherein the ECG microcontroller is further configured to:
analyze the second physiological data,
identify a second abnormal condition of the patient based upon the analyzed second physiological data,
determine a second significant physiological event based on the identified second abnormal condition of the patient,
transmit to the main controller in real-time, using the wireless communication connection, data corresponding to the second significant physiological event,
the main controller being configured to
receive the data corresponding to the second significant physiological event from the ECG module using the wireless communication connection (42);
transmit in real-time, using the first wireless communication protocol, data corresponding to the second significant physiological event; and
when the first wireless communication protocol is inoperable, transmit in real-time, using the second wireless communication protocol, the data corresponding to the second significant physiological event.

6. The physiological monitoring system of any preceding claim, wherein the main controller is further configured to:
store in the on-board memory (22, 30) one or more of the first physiological data, the second physiological data, the first abnormal condition, the second abnormal condition, the first significant physiological event and the second significant physiological event, optionally when the main module (7) is unable to transmit in real-time using the communication interface.

7. The physiological monitoring system of any of the preceding claims, wherein the ECG microcontroller (28) is further configured to:
generate one or more of an alarm signal and an alert signal, based on the identified one or more abnormal conditions.

8. The physiological monitoring system of any preceding claim, wherein the ECG module further comprises a data acquisition module (41) composed of a flexible polymer material with embedded circuitry and wherein the first plurality of electrodes (23) are formed in a flexible material integrated as a patch (40) having a top surface that is attachable to the data acquisition module and a bottom surface that is attachable to the patient.

9. The physiological monitoring system of claim 8, wherein the detachable precordial electrode array (53) includes a cable (50, 120) terminated with an in-line connector (51, 138) configured to be inserted into an external connection port of the data acquisition module, thereby establishing an electrical connection between the detachable precordial electrode array (53) and the ECG module.

10. The physiological monitoring system of claim 9, wherein:
the in-line connector (51, 138) of the cable (50, 120) includes a plurality of electrical contacts (93, 134), where each of the plurality of electrical contacts (93, 134) is configured to be aligned with a compression contact located in the external connection port.

11. The physiological monitoring system of claim 10, wherein:
the in-line connector (51, 138) of the cable (50, 120) further includes one or more of a seal (92, 135), a lock and a strain relief portion (90, 136), being configured to secure each of the plurality of electrical contacts (93, 134) is aligned with the compression contact located in the external connection port when the in-line connector (51, 138) is inserted into the external connection port.

12. The physiological monitoring system of any of the preceding claims, wherein one or more of the ECG module and the detachable precordial array further comprises adjustment slots and a location of each of the first plurality of electrodes (52) and/or the second plurality of electrodes (52) is adjustable within the adjustment slots.

## Patentansprüche

1. Physiologisches Überwachungssystem zum Bereitstellen von Überwachung eines Patienten, umfassend:
ein Elektrokardiogramm-(EKG-) Modul (15), das einen EKG-Mikrocontroller (28) und eine erste Mehrzahl von Elektroden (23) umfasst, die auf dem Patienten getragen werden, wobei der EKG-Mikrocontroller (28) mit der ersten Mehrzahl von Elektroden (23) gekoppelt ist, um erste physiologischen Daten zu empfangen, die durch die erste Mehrzahl von Elektroden (23) gesammelt werden;
ein Hauptmodul (7), das abnehmbar vom Patienten getragen wird und durch eine drahtlose Kommunikationsverbindung (42) mit dem EKG-Modul verbunden ist, wobei das Hauptmodul eine Hauptsteuerung, einen Platinenspeicher (22, 30) und eine Kommunikationsschnittstelle umfasst, die mit der Hauptsteuerung gekoppelt ist,
**dadurch gekennzeichnet, dass**:
die Kommunikationsschnittstelle ein erstes Drahtloskommunikationsprotokoll und ein vom ersten Drahtloskommunikationsprotokoll verschiedenes zweites Drahtloskommunikationsprotokoll umfasst;
der EKG-Mikrocontroller (28) ausgelegt ist zum:
Analysieren der ersten physiologischen Daten,
Identifizieren eines ersten anormalen Zustands des Patienten basierend auf den analysierten ersten physiologischen Daten;
Bestimmen eines ersten signifikanten physiologischen Ereignisses basierend auf dem identifizierten ersten anormalen Zustand des Patienten,
Senden von Daten, die dem ersten signifikanten physiologischen Ereignis entsprechen, unter Verwendung der drahtlosen Kommunikationsverbindung in Echtzeit an die Hauptsteuerung (7),
wobei die Hauptsteuerung ausgelegt ist zum:
Empfangen der Daten, die dem ersten signifikanten physiologischen Ereignis entsprechen, vom EKG-Modul unter Verwendung der drahtlosen Kommunikationsverbindung (42) ;
Senden von Daten, die dem ersten signifikanten physiologischen Ereignis entsprechen, in Echtzeit unter Verwendung des ersten Drahtloskommunikationsprotokolls und,
wenn das erste Drahtloskommunikationsprotokoll nicht funktionsfähig ist, Senden der Daten, die dem ersten signifikanten physiologischen Ereignis entsprechen, in Echtzeit unter Verwendung des zweiten Drahtloskommunikationsprotokolls.

2. Physiologisches Überwachungssystem nach Anspruch 1, wobei das erste Drahtloskommunikationsprotokoll WIFI (17) oder Bluetooth (14) entspricht.

3. Physiologisches Überwachungssystem nach einem der vorhergehenden Ansprüche, wobei das zweite Drahtloskommunikationsprotokoll einem Mobilfunknetzwerk entspricht.

4. Physiologisches Überwachungssystem nach einem der vorhergehenden Ansprüche, ferner umfassend eine abnehmbare präkordiale Elektrodenanordnung (53), die eine zweite Mehrzahl von Elektroden (52) umfasst, die an einer präkordialen Stelle des Patienten in der Nähe des EKG-Moduls getragen wird, wobei:
die zweite Mehrzahl von Elektroden (52) zum Sammeln von zweiten physiologischen Daten ausgelegt ist,
die abnehmbare präkordiale Elektrodenanordnung (53) zum Senden der zweiten physiologischen Daten durch eine drahtgebundene oder drahtlose Kommunikationsverbindung an das EKG-Modul ausgelegt ist.

5. Physiologisches Überwachungssystem nach Anspruch 4, wobei der EKG-Mikrocontroller ferner ausgelegt ist zum:
Analysieren der zweiten physiologischen Daten,
Identifizieren eines zweiten anormalen Zustands des Patienten basierend auf den analysierten zweiten physiologischen Daten;
Bestimmen eines zweiten signifikanten physiologischen Ereignisses basierend auf dem identifizierten zweiten anormalen Zustand des Patienten,
Senden von Daten, die dem zweiten signifikanten physiologischen Ereignis entsprechen, unter Verwendung der drahtlosen Kommunikationsverbindung in Echtzeit an die Hauptsteuerung,
wobei die Hauptsteuerung ausgelegt ist zum:
Empfangen der Daten, die dem zweiten signifikanten physiologischen Ereignis entsprechen, vom EKG-Modul unter Verwendung der drahtlosen Kommunikationsverbindung (42) ;
Senden von Daten, die dem zweiten signifikanten physiologischen Ereignis entsprechen, in Echtzeit unter Verwendung des ersten Drahtloskommunikationsprotokolls und,
wenn das erste Drahtloskommunikationsprotokoll nicht funktionsfähig ist, Senden der Daten, die dem zweiten signifikanten physiologischen Ereignis entsprechen, in Echtzeit unter Verwendung des zweiten Drahtloskommunikationsprotokolls.

6. Physiologisches Überwachungssystem nach einem der vorhergehenden Ansprüche, wobei die Hauptsteuerung ferner ausgelegt ist zum:
Speichern eines oder mehrerer der ersten physiologischen Daten, der zweiten physiologischen Daten, des ersten anormalen Zustands, des zweiten anormalen Zustands, des ersten signifikanten physiologischen Ereignisses und des zweiten signifikanten physiologischen Ereignisses in einem Platinenspeicher (22, 30), wobei optional die Kommunikationsschnittstelle verwendet wird, wenn das Hauptmodul (7) nicht in der Lage ist, in Echtzeit zu senden.

7. Physiologisches Überwachungssystem nach einem der vorhergehenden Ansprüche, wobei der EKG-Mikrocontroller (28) ferner ausgelegt ist zum:
Erzeugen eines oder mehrerer von einem Alarmsignal und einem Warnsignal basierend auf dem einen oder den mehreren identifizierten anormalen Zuständen.

8. Physiologisches Überwachungssystem nach einem der vorhergehenden Ansprüche, wobei das EKG-Modul ferner ein Datenerfassungsmodul (41) umfasst, das aus einem flexiblen Polymermaterial mit eingebetteten Schaltungen besteht, und wobei die erste Mehrzahl von Elektroden (23) in einem flexiblen Material ausgebildet ist, das als Pflaster (40) mit einer Oberseite, die am Datenerfassungsmodul angebracht werden kann, und einer Unterseite integriert ist, die am Patienten angebracht werden kann.

9. Physiologisches Überwachungssystem nach Anspruch 8, wobei die abnehmbare präkordiale Elektrodenanordnung (53) ein Kabel (50, 120) umfasst, das mit einem Inline-Verbinder (51, 138) abschließt, der dazu ausgelegt ist, in einen externen Verbindungsanschluss des Datenerfassungsmoduls eingeführt zu werden, um dadurch eine elektrische Verbindung zwischen der abnehmbaren präkordialen Elektrodenanordnung (53) und dem EKG-Modul herzustellen.

10. Physiologisches Überwachungssystem nach Anspruch 9, wobei:
der Inline-Verbinder (51, 138) des Kabels (50, 120) eine Mehrzahl von elektrischen Kontakten (93, 134) umfasst, wobei jeder der Mehrzahl von elektrischen Kontakten (93, 134) dazu ausgelegt ist, mit einem Kompressionskontakt ausgerichtet zu werden, der sich im externen Verbindungsanschluss befindet.

11. Physiologisches Überwachungssystem nach Anspruch 10, wobei:
der Inline-Verbinder (51, 138) des Kabels (50, 120) ferner eines oder mehrere von einer Dichtung (92, 135), einer Verriegelung und einem Zugentlastungsabschnitt (90, 136) umfasst, die zum Sichern jedes der Mehrzahl von elektrischen Kontakten (93, 134) ausgelegt sind, der mit dem Kompressionskontakt ausgerichtet ist, der sich im externen Verbindungsanschluss befindet, wenn der Inline-Verbinder (51, 138) in den externen Verbindungsanschluss eingeführt wird.

12. Physiologisches Überwachungssystem nach einem der vorhergehenden Ansprüche, wobei eines oder mehrere von dem EKG-Modul und der abnehmbaren präkordialen Anordnung ferner Anpassungsschlitze umfassen und eine Position jeder der ersten Mehrzahl von Elektroden (52) und/oder der zweiten Mehrzahl von Elektroden (52) innerhalb der Anpassungsschlitze angepasst werden kann.

## Revendications

1. Système de surveillance physiologique pour la surveillance d'un patient, comprenant :
un module d'électrocardiogramme (ECG) (15) comprenant un microcontrôleur ECG (28) et une première pluralité d'électrodes (23) portées par le patient, le microcontrôleur ECG (28) étant couplé à la première pluralité d'électrodes (23) pour recevoir des premières données physiologiques recueillies par la première pluralité d'électrodes (23) ;
un module principal (7) porté de manière amovible par le patient et connecté au module ECG par une connexion de communication sans fil (42), le module principal comprenant un dispositif de commande principal, une mémoire embarquée (22, 30), et une interface de communication couplée au dispositif de commande principal,
**caractérisé par** :
l'interface de communication comprenant un premier protocole de communication sans fil et un second protocole de communication sans fil différent du premier protocole de communication sans fil ;
le microcontrôleur ECG (28) étant configuré pour :
analyser les premières données physiologiques,
identifier un premier état anormal du patient sur la base des premières données physiologiques analysées,
déterminer un premier événement physiologique significatif sur la base du premier état anormal du patient identifié,
transmettre au dispositif de commande principal (7) en temps réel, au moyen de la connexion de communication sans fil, des données correspondant au premier événement physiologique significatif ;
le dispositif de commande principal étant configuré pour :
recevoir les données correspondant au premier événement physiologique significatif du module ECG au moyen de la connexion de communication sans fil (42) ;
transmettre en temps réel, au moyen du premier protocole de communication sans fil, des données correspondant au premier événement physiologique significatif, et
lorsque le premier protocole de communication sans fil est inopérant, transmettre en temps réel, au moyen du second protocole de communication sans fil, les données correspondant au premier événement physiologique significatif.

2. Système de surveillance physiologique selon la revendication 1, le premier protocole de communication sans fil étant conforme à WIFI (17) ou Bluetooth (14).

3. Système de surveillance physiologique selon l'une quelconque des revendications précédentes, le second protocole de communication sans fil étant conforme à un réseau cellulaire.

4. Système de surveillance physiologique selon l'une quelconque des revendications précédentes, comprenant en outre un réseau d'électrodes précordiales détachable (53) comprenant une seconde pluralité d'électrodes (52) portées dans un emplacement précordial du patient à proximité du module ECG,
la seconde pluralité d'électrodes (52) étant configurée pour recueillir des secondes données physiologiques,
le réseau d'électrodes précordiales détachable (53) étant configuré pour transmettre les secondes données physiologiques au module ECG par une connexion de communication câblée ou sans fil.

5. Système de surveillance physiologique selon la revendication 4, le microcontrôleur ECG étant en outre configuré pour :
analyser les secondes données physiologiques,
identifier un second état anormal du patient sur la base des secondes données physiologiques analysées,
déterminer un second événement physiologique significatif sur la base du second état anormal du patient identifié,
transmettre au dispositif de commande principal en temps réel, au moyen de la connexion de communication sans fil, des données correspondant au second événement physiologique significatif,
le dispositif de commande principal étant configuré pour
recevoir les données correspondant au second événement physiologique significatif du module ECG au moyen de la connexion de communication sans fil (42) ;
transmettre en temps réel, au moyen du premier protocole de communication sans fil, des données correspondant au second événement physiologique significatif ; et
lorsque le premier protocole de communication sans fil est inopérant, transmettre en temps réel, au moyen du second protocole de communication sans fil, les données correspondant au second événement physiologique significatif.

6. Système de surveillance physiologique selon l'une quelconque des revendications précédentes, le dispositif de commande principal étant en outre configuré pour :
stocker dans la mémoire embarquée (22, 30) une ou plusieurs des premières données physiologiques, des secondes données physiologiques, du premier état anormal, du second état anormal, du premier événement physiologique significatif et du second événement physiologique significatif, éventuellement lorsque le module principal (7) n'est pas apte à transmettre en temps réel au moyen de l'interface de communication.

7. Système de surveillance physiologique selon l'une quelconque des revendications précédentes, le microcontrôleur ECG (28) étant en outre configuré pour :
générer un ou plusieurs signaux parmi un signal d'alarme et un signal d'alerte, sur la base d'un ou plusieurs états anormaux identifiés.

8. Système de surveillance physiologique selon l'une quelconque des revendications précédentes, le module ECG comprenant en outre un module d'acquisition de données (41) composé d'un matériau polymère flexible avec des circuits intégrés et la première pluralité d'électrodes (23) étant formée dans un matériau flexible intégré sous la forme d'un patch (40) ayant une surface supérieure qui peut être fixée au module d'acquisition de données et une surface inférieure qui peut être fixée au patient.

9. Système de surveillance physiologique selon la revendication 8, le réseau d'électrodes précordiales détachable (53) comprenant un câble (50, 120) terminé par un connecteur en ligne (51, 138) configuré pour être inséré dans un port de connexion externe du module d'acquisition de données, établissant ainsi une connexion électrique entre le réseau d'électrodes précordiales détachable (53) et le module ECG.

10. Système de surveillance physiologique selon la revendication 9, le connecteur en ligne (51, 138) du câble (50, 120) comprenant une pluralité de contacts électriques (93, 134), où chacun de la pluralité de contacts électriques (93, 134) est configuré pour être aligné avec un contact de compression situé dans le port de connexion externe.

11. Système de surveillance physiologique selon la revendication 10, le connecteur en ligne (51, 138) du câble (50, 120) comprenant en outre un ou plusieurs éléments parmi un joint d'étanchéité (92, 135), un verrou et une partie de relâchement de contrainte (90, 136), qui sont configurés pour fixer chacun de la pluralité de contacts électriques (93, 134), étant aligné avec le contact de compression situé dans le port de connexion externe lorsque le connecteur en ligne (51, 138) est inséré dans le port de connexion externe.

12. Système de surveillance physiologique selon l'une des revendications précédentes, un ou plusieurs modules ECG et le réseau précordial détachable comprenant en outre des fentes de réglage, et un emplacement de chacune de la première pluralité d'électrodes (52) et/ou de la seconde pluralité d'électrodes (52) étant réglable à l'intérieur des fentes de réglage.
